Europäisches Patentamt

European Patent Office

Office européen des brevets

(11) Publication number: **0 292 656**
**A1**

(12) **EUROPEAN PATENT APPLICATION**

(21) Application number: **88103612.3**

(22) Date of filing: **08.03.88**

(51) Int. Cl.⁴: **G01N 33/577 , G01N 33/74 , G01N 33/68 , G01N 33/541 , G01N 33/543**

(30) Priority: **29.05.87 US 55812**

(43) Date of publication of application:
**30.11.88 Bulletin 88/48**

(84) Designated Contracting States:
**AT BE CH DE ES FR GB GR IT LI LU NL SE**

(71) Applicant: **MALLINCKRODT, INC.(a Missouri corporation)**
**675 McDonnell Boulevard P.O. Box 5840**
**St. Louis Missouri 63134(US)**

(72) Inventor: **Scott, Mitchell G.**
**12404 Matthews Lane**
**Sunset Hills Mo. 63127(US)**

(74) Representative: **Kraus, Walter, Dr. et al**
**Patentanwälte Kraus, Weisert & Partner**
**Thomas-Wimmer-Ring 15**
**D-8000 München 22(DE)**

(54) **Novel pair of monoclonal antibodies to insulin-like growth factor I permits immunometric assay for IGF-I.**

(57) A novel pair of complementary antibodies against Insulin-Like Growth Factor I (IGF-I) is unexpectedly isolated following intraperitoneal immunization without conjugating IGF-I to a carrier protein. The antibodies permit a highly effective immunometric (which could be radioimmunometric, enzymeimmunometric, luminescence or fluorescence immunometric) assay for IGF-I.

EP 0 292 656 A1

# NOVEL PAIR OF MONOCLONAL ANTIBODIES TO INSULIN-LIKE GROWTH FACTOR I PERMITS IMMUNOMETRIC ASSAY FOR IGF-I

## FIELD OF THE INVENTION

This invention relates to clinical methods for measuring serum Insulin-like growth Factor I (IGF-1) levels and to methods for isolating the monoclonal antibodies employed to effect such measurements.

## BACKGROUND OF THE INVENTION

Insulin-like growth factors (IGF's) are a family of closely related peptides that exert a variety of insulin-like and growth promoting effects on living cells, particularly mediating the growth promoting actions of growth hormone on skeletal and other body tissues. IGF-I (Somatomedin C) and IGF-II (Sometomedin A) are small proteins of approximately 7500 daltons molecular weight and contain approximately 62% amino acid homology with each other.

IGF-I is believed to be secreted at a variety of sites or cells in a variety of body tissues, rather than only in the liver as was conjectured during the initial investigations of this substance. Its levels in plasma can increase as growth hormone is released from the pituitary gland. Accordingly, IGF-I secretion is primarily regulated by growth hormone.

Serum IGF-I levels have been found to correlate well with growth status in growing animals and humans. Growth failure, for example, has been found to result from either (a) the incapacity to secrete growth hormone with a consequent deficiency of IGF-I, as in children with pituitary disease or (b) a peripheral resistance to IGF-I, as in female children with Turner's syndrome who grow at a subnormal rate despite IGF levels approaching normal. In addition, individuals with normal human growth hormone levels fail to produce optimal levels of IGF-I at times. A recent survey of IGF-I levels in over 500 pygmy individuals strongly suggest that IGF-I is the principal factor in regulating pubertal growth. IGF-I levels are, therefore, clinically important for assessing growth status in patients with growth abnormalities.

With growth hormone therapy, many hypopituitary children exhibit a rise of serum IGF-I concentrations to the normal range. In adults, the excessive production of growth hormone due to pituitary pathologies may lead to physical deformities such as acromegaly. This condition often goes undetected until the disfiguration becomes pronounced because of the difficulties in measuring growth hormone which has a half life of minutes and which is secreted in a pulsatile nature. Fortunately, it has been found that IGF-I levels are highly elevated before these structural changes are obvious, and therapy for these individuals can be initiated and successfully monitored using IGF-I determinations. Furthermore, unlike human growth hormone which exhibits acute fluctuations, IGF-I serum levels are stable, or change very gradually. Thus, IGF-I measurement can be of greater clinical value than direct measurements of human growth hormone since IGF-I more accurately reflects chronic growth status and eliminates the necessity of multiple or provocative growth hormone determinations for a review of the clinical utility of IGF-1 see W.H. Daughaday, et al., J. Clin. Endocrinal. Metab. 1987, 109:355, entitled "Serum Somatomedin Binding Proteins: Physiologic Significance and Interference in Radioligand Assay".

Nevertheless, routine measurement of IGF-I has not become common in clinical laboratories. Methods for measuring IGF-I have included in-vitro bioassays as described by W.H. Daughaday, et. al. in Volume 37 (1975) pages 93-109 of Methods In Enzymology in an article entitled "Measurement of Somatomedin By Cartilage In-Vitro". Additionally, radioreceptor assays have been employed as described in the Journal of Clinical Endocrinology and Metabolism, Volume 39, Pages 283-292 by R.N. Marshall et al., the Journal of Clinical Endocrinology and Metabolism, Volume 53, Pages 282-288 by Daughaday et al., and in the Journal of Clinical Endocrinology, Volume 24, Pages 267-278 by Baxter et al. These methods, however, are lengthy, technically complex and often present problems in interpretation. Thus, they clearly are not practical for routine use in clinical laboratories.

Polyclonal antisera specific for IGF-I have been found which permit competitive inhibition immunoassays. (See Journal of Clinical Investigations, Volume 60, Pages 648-657 by Furlanetto et al.). These techniques are based upon the formation of a complex between the IGF-I being assayed, IGF-I labeled by a radioactive element $^{125}I$ and polyclonal antiserum. The IGF-I being tested competes with a known quantity of labeled IGF-I for a limited quantity of polyclonal antiserum binding sites. The amount of labeled IGF-I bound to the polyclonal antiserum is inversely proportional to the amount of IGF-I in the sample. Thus, detection and/or quantitative analysis after separation of the complexed labeled IGF-I from uncomplexed labeled IGF-I provides the basis for the measurement. These assays require multiple steps, lengthy incubations, difficult separation of bound from free ligand and are so cumbersome that they are of poor utility for routine clinical laboratory use.

Although monoclonal antibodies have been developed against IGF-I by conjugating the IGF-I to large molecules, followed by subsequent immunization, and fusion with myeloma cells resulting in in-vitro production of the hybridoma, these prior art monoclonal antibodies have not completely corrected the problem of cross-reactivity. Journal of Clincal Endocrinology and Metabolism, Volume 54, Pages 474-476 by Baxter et al., Biochemical Journal, Volume 233, Pages 215-221 by Read et al., and FEBS Letters, Volume 149, Page 109 by Laubli et al.

Radioimmunoassays for IGF-I are interfered with by the presence of IGF-binding protein complexes within the serum to be measured. These complexes must be disassociated prior to serum assay to accurately measure the total IGF-I. Various extraction procedures including acidification, acid-ethanol mixtures, acid chromatography and proteolytic cleavage have been employed in attempts to solve this problem. However, these extraction procedures are quite tedious and necessitate evaporation and reconstitution, or neutralization of the extracted samples.

U.S. Patent 4,376,110 by Gary David discloses simplified "two-site" or "sandwich" radioimmunometric assay techniques in which the antibody is labeled rather than the antigen to be measured. Such assays employ a quantity of unlabeled monoclonal antibody bound to a solid support and a quantity of soluble monoclonal antibody bearing a radioactive or enzyme conjugated label. The technique permits detection and/or a quantitative measurement of the amount of a complex formed between the solid phase antibodies, the antigens, and the labeled soluble antibodies.

However, this procedure requires a complementary set of antibodies which bind to different sites on the antigen. To produce such complementary antibodies the larger molecular-size antigens such as insulin itself, thyroid stimulating hormone, gamma globulins, allergens, viruses, virus subunits, bacteria, toxins such as those associated with tetanus and with animal venoms have proven quite successful due to the availability of a variety of different antigenic sites on the larger molecules. Examples of such success were carcinoembryonic antigen, Hepatitis A & B, Hepatitis Non-A/Non-B, IgE and alphafetoprotein. However, such techinques have not been previously adapted for smaller molecular weight protein antigens of less than 8,000 dalton units and particularly IGF-I. Monoclonal antibodies to IGF-I of sufficient complementary nature and of non-reactive IGF-II cross reactivity which allow an IRMA configuration have not been heretofore developed.

A simplified radioimmunometric procedure which can solve the problems of prior art clinical assays for IGF-I and simplify the extraction procedure of IGF-I antiserum would be a substantial advancement in the art.


## SUMMARY OF THE INVENTION


Accordingly, one object of the present invention is to provide an improved method for the immunoradiometric assay of IGF-I.

More specifically, an object of the present invention is to provide an isolation of monoclonal antibodies to IGF-I which permits a more rapid and simultaneous incubation radioimmunometric assay technique.

Another object of the present invention is to provide a method of using these novel monoclonal antibodies in a more sensitive immunoradiometric assay technique for IGF-I.

Yet another object of the present invention is to provide a procedure for extracting IGF-I serum in order to conduct an assay without evaporation and reconstitution, or neutralization of the extracted samples.

According to the present invention, a simultaneous incubation monoclonal antibody based immunoradiometric assay for IGF-I is provided having the advantages of short incubation, ease of performance and less than 1.2% cross-reactivity with IGF-II. The sensitivity of this assay also allows unexpected simplification of the extraction procedure. This novel immunoradiometric assay specific for IGF-I should provide a simple routine measurement of IGF-I in clinical laboratories.

## 0 292 656

DETAILED DESCRIPTION OF THE PREFERRED EMBODIMENTS

In accordance with the present invention, a novel pair of antibodies to spatially distinct sites (complementary) on IGF-I are obtained through intraperitoneal immunization of mice with pure, non-conjugated IGF-I.

Purified, bioactive recombinant IGF-I may be prepared preferably from specifically constructed E. coli strains as described by Buell et al. in Nucleic Acids Research, Volume 13, Pages 1923 through 1937 entitled "Optimizing the Expression in E. coli of a Synthetic Gene Encoding Somatomedin C (IGF-I)" and as described by Burleigh et al. in the Journal of Biological Chemistry submitted for publication in 1986 entitled "Characterization of Two Forms of Somatomedin C Produced by Recombinant Bacteria". Purity and homogeneity of the recombinant IGF-I preparation were established by standard methods of protein characterization, including SDS-polyacrylamide gel electrophoresis, isoelectric focusing, amino acid analysis, size exclusion gel chromatography, and reversed-phase HPLC (high performance liquid chromatography). The purified recombinant IGF-I is found to bind specifically to IGF receptors in a human placental membrane radioreceptor assay. The binding is comparable to that of natural, purified IGF-I in terms of affinity. The preparation is immunologically identical to endogenous IGF-I as previously shown by Baxter et al. in their 1987 article; Clinical Chemistry 33:544 entitled, "Comparison of Natural and Recombinant DNA Derived Human Insulin-Like Growth Factor One and Radiological Assays". The preparation is biochemically identical to endogenous IGF-I with the exception of a methionine leader residue at the N-terminus.

Despite the fact that IGF-I molecules are so small that they may be considered poorly immunogenic without coupling to carrier proteins such as ovalbumin or keyhole limpet hemocyanin, the non-conjugated IGF-I at for example a total of 25 micrograms (ug) per animal, is used to immunize mice intraperitoneally with Freund's complete adjuvant and boosted on days 14, 28 and 38 with the same amount of IGF-I in incomplete Freund's adjuvant. Standard fusion methods of Shulman et al., Nature 1978 276:269, are performed three days after the last boost. Selected cell lines may then be cloned by limiting dilution after which hybridoma cell cultures are expanded and injected into retired breeders for ascites production. Monoclonal antibodies were purified for further use by precipitation in 50% saturated ammonium sulfate followed by high performance liquid chromatography ion-exchange chromatography. They could then be iodinated by modification of the well-known Hunter-Greenwood Chloramine T method or they could be used to coat surfaces.

The purified monoclonal antibodies discovered in this way were surprisingly found to be complementary and are identified as (1) 2D12.1, having less than 1.2% cross-reactivity with IGF-II and, (2) 6B1.1. No previous investigation has reported such a "complementary" set of monoclonal antibodies (Mabs). The surprising isolation of these complementary antibodies is believed to stem from the non-conjugated form of IGF-I used for immunization. Although this occurrence is not completely understood, one rationalization is that despite the small molecular size of IGF-I being less immunogenic than if it were coupled to a carrier protein, the ability or the probability of identifying monoclonal antibodies to spatially distinct epitopes on the IGF-I itself was enhanced by immunizing the IGF-I without conjugating or coupling it to a carrier protein. Previous immunizations with carrier protein conjugated IGF-I may have hindered accessibility of some epitopes or antigenic sites, to the immune system of the mouse.

The two monoclonal antibodies 6B1.1 and 2D12.1 were found to bind to separate epitopes on IGF-I as neither antibody in the liquid phase inhibited recombinant IGF-I binding by the other solid-phase antibody. Each antibody totally inhibited its own solid phase binding. Both antibodies have high binding affinities, for example, $Ka = 2 \times 10^9$ L/Mole for 6B1.1 and $6 \times 10^8$ L/Mole for 2D12.1.

The IGF-I specific immunoradiometric assay may be performed in accordance with the following steps of the invention.

Of these two monoclonal antibodies preferably 6B1.1 is passively absorbed on a solid surface, and preferably the inner surface of polystyrene tubes. Other surfaces may be for example polystyrene beads, polystyrene or polyvinyl microtiter plates, polyethylene tubes, or latex beads. The amount of coating antibody may be between 500 ng (nanograms) and 10 ug (micrograms).

The soluble liquid phase Mab's 2D12.1 could readily be coupled to an enzyme such as horseradish peroxidase, alkaline phosphatase or B-Galactosidase, etc. to perform an enzyme immunoassay (EIA) version of this invention or to a luminogenic compound such as luminol, isoluminol, acridinium and phenanthridinium esters and their derivatives to perform a luminescent immunoassay version (LIA) of the invention or to other labelling compounds such as fluorophores, etc.

This second of the monoclonal antibodies, is labeled with any of the several labels used in the prior art in immunometric assays but preferably iodine 125 and is dissolved in a solution containing, generally, 10-150 mM salt, 0.0-1% bovine serum albumin or other non-specific protein preferably sodium phosphate neutralized to a pH of from 6.5 to 8.0 preferably 7.4 in sodium chloride, and preferably containing heparin and a chelating agent such as, preferably ethylenediamine tetracetic acid. The solution is added to the polystyrene or other plastic tubes coated with the first monoclonal antibody and the serum to be tested is immediately added to the tube in an amount of for example 25 ul (microliters) to 100 ul, preferably 25 ul.

The fluid such as urine, saliva, tissue cultures, supernatants but preferably serum to be tested in accordance with this invention should be extracted prior to testing to eliminate interference from the IGF-binding proteins. Such extraction is quite simplified as compared to prior art extractions. The Prior art necessitated neutralization or alternatively evaporation and reconstitution of the extracted samples prior to use in radioimmunoassays or in some cases. The current invention negates these latter steps. Binding proteins may be eliminated in accordance with this invention by extracting the serum with acid ethanol incubated at room temperature for 30 minutes. Samples may then be centrifuged for 10 minutes and a fraction of the supernatant is used directly in the assay without the prior art steps of evaporation or neutralization. Preferably 0.2M Formic Acid in 85% ETOH or from 0.1N to .5M of Formic Acid or HCl in from 70-95% ETOH.

The extracted serum or, if desired, a serum standard after being added to the tube may be incubated at temperatures of from 20°C to 40°C degrees, preferably minutes, preferably about three hours. Tubes may then be decanted, washed and the radioactivity level counted. The IGF-I levels are determined by comparing the $^{125}$I counts bound in sample tubes directly to a standard curve. The standard curve may be described as a plot of counts of $^{125}$I-2D12.1 Mab bound in the assay versus IGF-I concentration in the standards assayed. The assay is linear from 0-800 ug/L IGF-I and does not exhibit a "hook" effect (high dose inhibition) in concentrations up to 4 mg/L. No cross-reactivity is observed with either insulin or pro-insulin. IGF-II exhibited less than 1.6% cross-reactivity. Sensitivity (minimal detectable dose) of the assay is 5 ug/L based on count rates twice that of background. Linear regression analysis of this standard curve demonstrates a correlation coefficient of 0.99 and coefficient of variance range from 3.3% to 10% for the different standard concentrations. (n = 5) Coefficient of variance (C.V.) may be defined as the standard deviation divided by the mean value.

The invention will be further illustrated and other embodiments will become evident from the following examples which are not intended to limit the scope of the invention.

In the examples, the following abbreviations will appear:

BSA = bovine serum albumin
Ig = immunoglobin
L = liter
MAb = monoclonal antibody
Mg = milligram
MIg = mouse immunoglobin
ml = milliliter
ng = nanogram
PBS = phosphate buffered saline
ug = microgram
ul = microliter


## EXAMPLE I


## IDENTIFICATION OF IGF-I BINDING MONOCLONAL ANTIBODIES

10-14 days following fusion, supernatants fron primary hybridoma culture wells were harvested and screened for the ability to bind $^{125}$I-IGF-I as follows. Polyvinyl chloride microtiter wells were coated with 150 ml of 2 ug/ml affinity purified goat anti-mouse immunoglobin (MIg) in phosphate buffered saline (pH 7.4). Wells were blocked with 10 g/L bovine serum albumin for 30-60 minutes. 150 ul of primary hybridoma culture well supernatants were transferred to the anti-mouse Ig coated plates. If mouse monoclonal antibodies were present in the culture supernatant they are bound by the solid phase goat anti-MIg on the microtiter wells. These supernatants were incubated in the wells overnight at room temperature. Following this incubation the supernatants were removed, leaving behind any bound MAb, and washed three times

with $H_2O$. $^{125}I$-IGF-I, 100,000 cpm in 150 ul of 10 g/L BSA, 10 mM phosphate buffered saline, ph 7.4, was then added to each microtiter well and incubated for 3 hours at room temperature. Any of the MAb's captured on the microtiter wells with specificity for IGF-I would bind the $^{125}I$-IGF-I and be identified by high cpm rates when the wells were washed, counted and separated. Actual data from the initial screening assay which demonstrated that MAb's 2D12.1 and 6B1.1 bound IGF-I is shown in Table 1. The names 2D12.1 and 6B1.1 are arbitrary and designate the wells in which they were originally identified.

2). DETERMINATION OF WHETHER MAb's 6B1.1 and 2D12.1 BOUND SPATIALLY DISTINCT EPITOPES ON IGF-I.

The ability of different MAb's to simultaneously bind distinct epitopes on IGF-I (complementarity) was determined by an inhibition assay in which two MAb's were allowed to compete for soluble $^{125}I$-IGF-I. Briefly, $10^5$ cpm of IGF-I was incubated with 10ug of a particular MAb in 150ul of 1% BSA,PBS for 1 hr. This was then added to microtiter wells previously coated with the same MAb or with other MAb's. Complementarity of the MAb's was assessed by the amount of inhibition of $^{125}I$-IGF-I binding to the solid phase antibody by the liquid phase antibody.

The approximate affinity of these monoclonal antibodies was determined using a modification of the Sips equation as previously described in the article by Nahm et al., J. Immunol. 1977, 119:301 entitled, "A New Method of Applying the Sips Equation".

The two MAb's, 6B1.1 and 2D12.1, were shown to bind spatially distinct epitopes as neither antibody in the liquid phase inhibited met-hIGF-I binding by the other solid phase antibody (Table 3). Each antibody completely inhibited its own solid phase binding. Both antibodies were shown to have high binding affinities; $Ka = 2x10^9$ L/Mole for 6B1.1 and $6x10^8$ L/Mole for 2D12.1.

## EXAMPLE II

## CHARACTERIZATION OF SELECTED IGF-I SPECIFIC MAb's

MAb's identified as binding IGF-I in the above screening assay were further characterized. Because of the uniqueness of MAb's 6B1.1 and 2D12.1 this example will be limited to these MAb's.

## 1.) DETERMINATION OF CROSS-REACTIVITY OF MAb's 6B1.1 AND 2D12.1 WITH PROTEINS RELATED TO IGF-I.

MAb Characterization: The specificity of these antibodies was determined in a competitive inhibition microtiter plate radioimmunoassay as previously described by Perlmutter et al., J. Immunol., 1978, 121:566 entitled, "Subclass Restriction of Murine Anticarbohydrate Antibodies". Briefly, 96-well flexible, polyvinyl chloride microtiter plates (Falcon) were coated with optimal dilutions (0.5-2 mg/ml) of purified anti-IGF-I Mab's 6B1.1 or 2D12.1 and then blocked with bovine serum albumin (10 mg/ml) in phosphate buffered saline (PBS) (pH 7.4). Samples tested as inhibitors were added and three fold serial dilutions were performed within the plate. $10^5$ cpm of $^{125}I$-IGF-I was added to each well and incubated at room temperature for 4-6 hrs. Inhibition by samples was compared to IGF-I standards and the cross-reactivity was estimated.

Specificity of these two antibodies was determined by inhibition RIA as described above. Table 2 shows the concentration of various proteins which inhibited 50% of maximal binding of $^{125}I$-IGF-I by the two antibodies and also shows the apparent cross-reactivities. Human somatotropin and prolactin exhibited no detectable cross-reactivity with either antibody. MAb 6B1.1 exhibits 21% cross-reactivity with IGF-II, while MAb 2D12.1 has less than 1.2% cross-reactivity with IGF-II in this assay.

## EXAMPLE III

## IGF-I ASSAY STANDARD CURVE AND DETERMINATION OF CROSS REACTIVITY OF THE IGF-I IRMA INVENTION

The IGF-I specific IRMA assay was developed with modifications of previously described methods for MAb's in the article by Sevier et al., Clinical Chemistry, 1981, 27:1797, entitled "Monoclonal Antibodies in Clinical Immunology". The assay is performed by adding $2 \times 10^5$ cpm of [$^{125}$ I]-MAb 2D12.1, in 1 ml of 100mM sodium phosphate, 150 mM NaCl, pH 7.4, 0.3U/ml heparin, 1.0 ml/L Tween 20, 1g/L EDTA, pH 7.4, to a polystyrene tube (Maxisorb, NUNC) passively absorbed with 2.5 ug of MAb 6B1.1. 25ul of extracted serum or standard is then added to the tube and incubated at 37° C for 3 hours. Tubes are then decanted, washed three times with water and counted. IGF-I levels are determined by comparing counts bound in sample tubes to the standard curve.

Protein Standards: IGF-I standards were prepared in 10mM phosphate, 150mM NaCl, pH 7.4, (PBS) containing 10 g/L bovine serum albumin (BSA) and 20 Units/ml heparin. All BSA used in these procedures was pre-screened to assure that no IGF-I was present.

IGF-II was kindly provided by Dr. D. Harlein, Biogen S.A. Purified human prolactin and somatotropin were purchased from Calbiochem (LaJolla, CA). Human insulin was purchased from Sigma Chemical (St. Louis, Mo.) and human proinsulin was kindly provided by Dr. H. Tager, University of Chicago.

These other proteins were prepared in the same standard buffer used for the IGF-I standards.

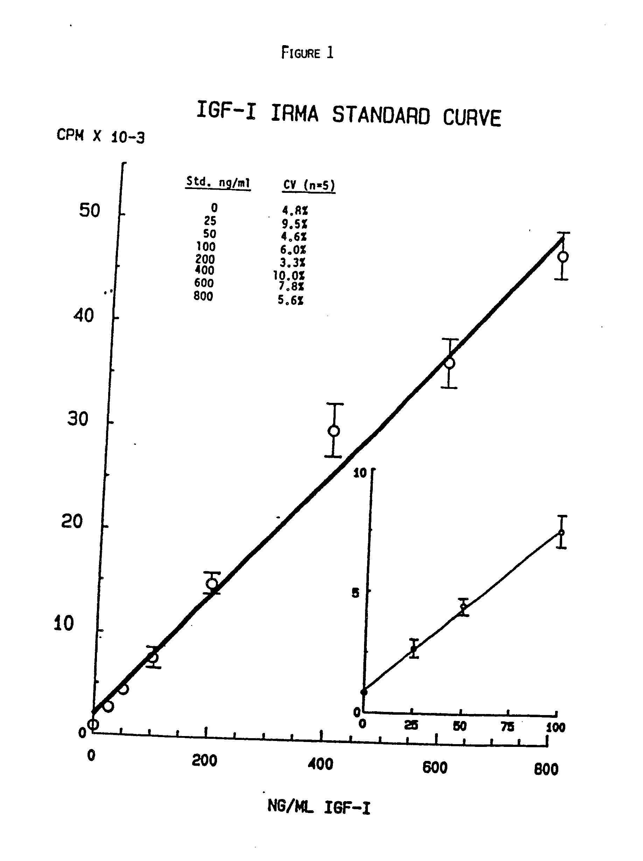

Figure 1. and Table 4 depict a representative standard curve obtained with the IGF-I IRMA assay. The assay is essentially linear from 0-800 ug/L with a sensitivity of 10 ug/L for serum samples. No decrease in count rates was observed with standards as high as 4 mg/L (not shown). No detectable cross-reactivity was seen with, insulin or pro-insulin and IGF-II exhibited 1.6% cross reactivity in the IGF-I IRMA assay (Table 4) . Linear regression analysis of this standard curve demonstrated a correlation coefficient of 0.99 and C.V's ranged from 3.3% to 10% at the different standard concentrations.

## EXAMPLE IV

## IRMA ASSAY PERFORMANCE ON SERUM SAMPLES

Serum samples were obtained from patients with disorders of growth from the Radioimmunoassay Core Laboratory in the Diabetes Research and Training Center at Washington University, School of Medicine, St. Louis, Missouri. IGF-I determinations had been performed in that laboratory, after acid-ethanol extraction using a previously published double antibody RIA as described in Daughaday et al., J. Clin Endo. Metab., 1980 51:781.

IRMA Assay Performance: Recoveries of IGF-I added to five patient sera ranged from 75-103% as shown in Table 5. Intra-assay precision studies with 5 patient sera resulted in CV's of 2.5% to 7.8% (Table 6). Interassay precision with these same samples resulted in CV's of 1.8% to 14% (Table 7).

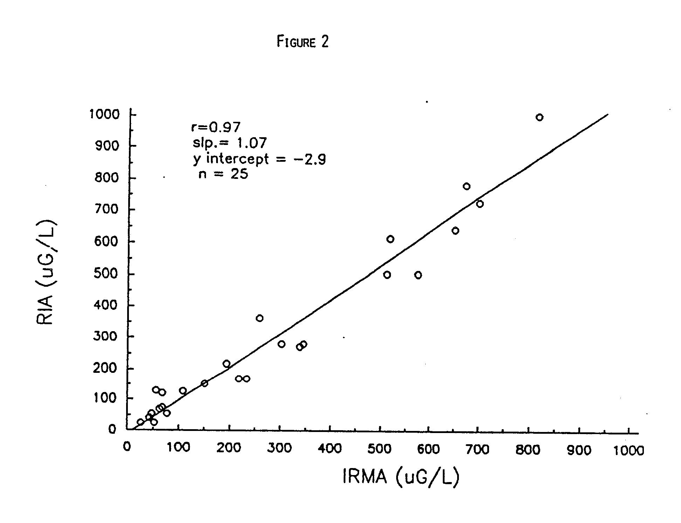

Twenty-five patient sera with RIA values covering a wide analytical range (25-1008 ug/L) were run in both the IRMA and RIA (Figure 2). Linear regression analysis of the values obtained from the RIA and IRMA show excellent correlation, r = 0.97 and no significant bias, slope = 1.07 (y = 1.07x + 2.9).

## TABLE 1

| HYBRIDOMA SUPERNATANT | $^{125}$I-IGF-I BOUND[a] |
|---|---|
| 6B1.1 | 33,356 |
| 2D12.1 | 55,341 |
| [b]MEDIA | 1,143 |
| [c]IRRELEVANT MAb To THYROTROPIN | 1,267 |

[a] Cpm bound in microtiter well coated with Goat anti-MIg, followed by hybridoma culture supernatant as described in text.

[b] Culture media as non-specific binding control.

[c] MAb to Thyrotropin (TSH 7D3.2) as non-specific, irrelevant MAb control.

## TABLE 2

### SPECIFICITY OF ANTI-IGF-I MAb'S

| Inhibitor | [a](mg/L) 2D12.1 | 6B1.1 | [b] Apparent % Cross-Reactivity 2D12.1 | 6B1.1 |
|---|---|---|---|---|
| IGF-I | 6.0 | 10.2 | 100 | 100 |
| IGF-II | > 500 | 47.6 | < 1.2 | < 2 |
| hu Prolactin | > 500 | > 500 | < 1.2 | < 2 |
| hu Somatotropin | > 500 | > 500 | < 1.2 | < 2 |

[a] Concentration required to produce 50% $B/B^0$ in microtiter plate inhibition assay as described in materials and methods.

[b] Calculated from the concentration of met-IGF-I resulting in 50% $B/B^0$ divided by the concentration of the other protein which also resulted in 50% $B/B_0$.

$$B/B_0 = \frac{\text{Bound in presence of inhibitor}}{\text{Bound in absence of any inhibitor (i.e. maximum binding)}}$$

## TABLE 3

## cpm IGF-I BOUND

| [b]SOLUBLE MAb | [a]SOLID PHASE MAb | | | |
|---|---|---|---|---|
| | 6B1.1 | 2D12.1 | [c]MEDIA | [d]PROLACTIN 1B2.1 |
| 6B1.1 | 1,431 | 39,672 | 1,412 | 1,531 |
| 2D12.1 | 18,381 | 1,312 | 1,583 | 1,287 |
| MEDIA | 33,338 | 45,624 | 1,621 | 1,812 |
| [d]PROLACTIN 1B2.1 | 31,020 | 43,015 | 1,232 | 1,539 |

a   Purified MAb Adsorbed to Surface of Microtiter Well.

b   150 MAb Pre-Incubated with 100,000 cpm of $^{125}$I-IGF-I
     and Then Added to MAb Coated Micotiter Wells.

c   Hybridoma Culture Media Control for Non-Specific Binding.

d   Non-Specific MAb Control (MAb to Human Prolactin).

## TABLE 4

### IGF-I STANDARD CURVE AND CROSS-REACTIVITY STUDY

| IGF-I STANDARD ug/L | cpm $^{125}$I-2D12.1 BOUND | [a]IRMA CROSS-REACTIVITY |
|---|---|---|
| 0 | 834 | |
| 25 | 2,662 | |
| 50 | 4,439 | |
| 100 | 7,586 | |
| 200 | 14,910 | |
| 400 | 30,004 | |
| 600 | 36,812 | |
| 800 | 47,440 | |
| 4000 | 54,208 | |
| **PROINSULIN** | | |
| 10 mg/L | 929 | 0.001% |
| 2.5 mg/L | 867 | 0.002% |
| 1.0 mg/L | 848 | 0.01% |
| **INSULIN** | | |
| 100 mg/L | 884 | 0.0005% |
| 50 mg/L | 882 | 0.0001% |
| **IGF-II** | | |
| 1100 ug/L | 2,075 | 1.55% |
| 550 ug/L | 1,555 | 1.79% |
| 225 ug/L | 1,166 | 1.63% |

a  Calculated by linear regression.

## TABLE 5

### RECOVERY OF EXOGENOUS IGF-I

| Serum Sample No. | [a]Value | [b]Expected Value | Observed Value |
|---|---|---|---|
| 5513 | 287 | 487 | 477 (95%) |
| 5519 | 75 | 275 | 280 (103%) |
| 5523 | 77 | 277 | 260 (92%) |
| 5540 | 83 | 283 | 272 (95%) |
| 5532 | 168 | 368 | 319 (75%) |

[a]  Value obtained in IRMA in same run as recovery experiment performed.

[b]  Value expected following addition of 200ng IGF-I to 1 mL of serum.

## TABLE 6

### INTRAASSAY PRECISION

| Sample No. | n | (ug/L) | C.V. |
|---|---|---|---|
| 4744 | 5 | 585 + 14 | 2.5% |
| CB | 5 | 143 + 6.6 | 4.6% |
| 5532 | 5 | 228 + 18 | 7.8% |
| 5513 | 5 | 255 + 11 | 4.7% |
| 5519 | 5 | 88 + 5.2 | 5.9% |

**Claims**

1. An immunometric assay for measuring IGF-I in fluids comprising

(a) coating a solid phase immunometric assay surface with either of the uniquely complementary monoclonal antibodies 6B1.1 or 2D12.1;

(b) directly adding, to the system, acid-ethanol extracted fluid samples, which have not been evaporated and reconstituted or neutralized.

(c) adding, simultaneously with the sample addition, the labeled complementary second monoclonal antibody to the said solid-phase surface to form a complex binding

(i) whatever IGF-I is present,

(ii) the first immobilized monoclonal antibody, and (iii) the second labeled complementary antibody.

(d) simultaneously incubating the mixture for not more than three hours;

(e) washing the solid phase support to extract residual unreacted labelled antibodies and

(f) measuring the concentration of IGF-I bound in the complex by counting and comparing the counts directly to predetermined standard curves for IGF-I.

2. A method for isolating a complementary pair of monoclonal antibodies to IGF-I comprising intraperitoneal immunization without conjugating IGF-I to a carrier protein;

whereby monoclonal antibodies 6B1.1 and 2D12.1 are screened, the 2D12.1 having less than 1.2% reactivity to IGF-II.

3. The method of Claim 1 wherein the first monoclonal antibody coated to the solid surface is 6B1.1 and the labeled second antibody is 2D12.1.

4. The method of Claim 1 wherein said labeling is radioactive.

5. The method of Claim 1 wherein said labeling is enzymatic.

6. The method of Claim 1 wherein said labeling is luminescent.

7. The method of Claim 1 wherein said labeling is fluorescent.

8. A pair of complementary monoclonal antibodies to IGF-I suitable for immunometric clinical assays and identified following intraperitoneal immunization with IGF-I that is not conjugated to a carrier protein and identified as 6B1.1 and 2D12.1.

FIGURE 1

IGF-I IRMA STANDARD CURVE

FIGURE 2

RIA (uG/L) vs IRMA (uG/L)

r=0.97
slp.= 1.07
y intercept = −2.9
n = 25

European Patent Office

**EUROPEAN SEARCH REPORT**

Application number

| | DOCUMENTS CONSIDERED TO BE RELEVANT | | EP 88103612.3 |
|---|---|---|---|

| Category | Citation of document with indication, where appropriate, of relevant passages | Relevant to claim | CLASSIFICATION OF THE APPLICATION (Int. Cl 4) |
|---|---|---|---|
| P,A | CHEMICAL ABSTRACTS, vol. 107, no. 13, September 28, 1987, Columbus, Ohio, USA<br><br>L.E.UNDERWOOD "Use of plasma somatomedin C/insulinlike growth factor I (Sm-C/ICF-I). Determinations in clinical medicine" page 78, column 2, Abstract-no. 109 531c<br><br>& Horumon to Rinsho 1987, 35(3), 355-9<br><br>-- | 1 | G 01 N 33/577<br>G 01 N 33/74<br>G 01 N 33/68<br>G 01 N 33/541<br>G 01 N 33/543 |
| P,A | CHEMICAL ABSTRACTS, vol. 106, no. 23, June 8, 1987, Columbus, Ohio, USA<br><br>T.INOUE et al. "Determination of insulin-like growth factor-I in normal subjects and in patients with growth hormone disorders by radio-immunoassay using biosynthetic homologous peptide" page 85, column 1, Abstract-no. 189 201n<br><br>& Endocrinol. Jpn 1986, 33(6), 919-27<br><br>-- | 1 | |
| | | | TECHNICAL FIELDS SEARCHED (Int. Cl.4) |
| A | CHEMICAL ABSTRACTS, vol. 104, no. 19, May 12, 1986, Columbus, Ohio, USA<br><br>SHINOGI AND CO., LTD "Octadecapeptide for determination of insulin-like growth factor I." page 712, column 1, Abstract-no. 168 854w<br><br>& Jpn. Kokai Tokkyo Koho JP 60,109,599 (85,109,599)<br><br>-- | 1,2 | G 01 N 33/00 |

The present search report has been drawn up for all claims

| Place of search | Date of completion of the search | Examiner |
|---|---|---|
| VIENNA | 02-08-1988 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

<table>
<tr><td colspan="3" align="center"><strong>DOCUMENTS CONSIDERED TO BE RELEVANT</strong></td><td align="center">-2-<br>EP 88103612.3</td></tr>
<tr><td>Category</td><td align="center">Citation of document with indication, where appropriate,<br>of relevant passages</td><td>Relevant<br>to claim</td><td align="center">CLASSIFICATION OF THE<br>APPLICATION (Int. Cl 4)</td></tr>
<tr><td>A</td><td>CHEMICAL ABSTRACTS, vol. 106, no.<br>13, March 30, 1987, Columbus, Ohio,<br>USA<br><br>F.C.BUONOMO et al. "Determination of<br>insulin-like growth factor I (IGF1)<br>and IGF binding protein levels in<br>swine"<br>page 91, columns 1,2, Abstract-no.<br>96 311h<br><br>& Domest. Anim. Endocrinol. 1987,<br>   4(1), 23-31<br><br>--</td><td>1</td><td></td></tr>
<tr><td>A</td><td>CHEMICAL ABSTRACTS, vol. 99, no. 23,<br>December 5, 1983, Columbus, Ohio,<br>USA<br><br>J.ZAPF "Determination of insulin-<br>like growth factors: a survey of<br>methods"<br>page 107, column 2, Abstract-no.<br>187 812a<br><br>& Insulin-Like Growth Factors,<br>   Somatomedins, Proc. Symp. 1982<br>   (Pub. 1983), 133-8<br><br>--</td><td>1</td><td align="center">TECHNICAL FIELDS<br>SEARCHED (Int. Cl.4)</td></tr>
<tr><td>A</td><td>CHEMICAL ABSTRACTS, vol. 97, no. 17,<br>October 25, 1982, Columbus, Ohio,<br>USA<br><br>T.TSUSHIMA "Determination of insulin-<br>like growth factor"<br>page 89, column 1, Abstract-no.<br>138 764m<br><br>& Kenkyu Nenpo-Seicho Kagaku Kyokai<br>   1980 (Pub. 1981) 4, 85-90<br><br>--</td><td>1</td><td></td></tr>
</table>

The present search report has been drawn up for all claims

| Place of search | Date of completion of the search | Examiner |
|---|---|---|
| VIENNA | 02-08-1988 | SCHNASS |

| | **DOCUMENTS CONSIDERED TO BE RELEVANT** | | |
|---|---|---|---|
| **Category** | Citation of document with indication, where appropriate, of relevant passages | Relevant to claim | CLASSIFICATION OF THE APPLICATION (Int. Cl 4) |
| A | CHEMICAL ABSTRACTS, vol. 95, no. 25, December 21, 1981, Columbus, Ohio, USA<br><br>J.ZAPF et al. "Radioimmunological determination of insulinlike growth factors I and II in normal subjects and in patients with growth disorders and extrapancreatic tumor hypoglycemia"<br>page 385, column 1, Abstract-no. 218 639y<br><br>& J.Clin,Invest. 1981, 68(5), 1321-30<br><br>---- | 1 | |
| | | | TECHNICAL FIELDS SEARCHED (Int. Cl.4) |

The present search report has been drawn up for all claims

| Place of search | Date of completion of the search | Examiner |
|---|---|---|
| VIENNA | 02-08-1988 | SCHNASS |